**Europäisches Patentamt**

**European Patent Office**

**Office européen des brevets**

(19)

(11) Veröffentlichungsnummer: **0 288 895 B1**

(12)

# EUROPÄISCHE PATENTSCHRIFT

(45) Veröffentlichungstag der Patentschrift:
24.07.91 Patentblatt 91/30

(51) Int. Cl.⁵: **A61K 31/44,** A61K 31/21,
A61K 31/345, A61K 47/08

(21) Anmeldenummer: 88106348.1

(22) Anmeldetag: 21.04.88

(54) Arzneimittelformulierung.

(30) Priorität: 30.04.87 DE 3714402

(43) Veröffentlichungstag der Anmeldung:
02.11.88 Patentblatt 88/44

(45) Bekanntmachung des Hinweises auf die
Patenterteilung:
24.07.91 Patentblatt 91/30

(84) Benannte Vertragsstaaten:
AT BE CH DE ES FR GB GR IT LI LU NL SE

(56) Entgegenhaltungen:
EP-A- 0 061 806
EP-A- 0 175 671
EP-A- 0 182 635
EP-A- 0 230 226
DE-A- 1 792 448
DE-A- 1 808 922
DE-A- 3 315 805
GB-A- 970 027
LU-A- 65 929

(73) Patentinhaber: Kali-Chemie Pharma GmbH
Hans-Böckler-Allee 20 Postfach 220
W-3000 Hannover 1 (DE)

(72) Erfinder: Block, Jürgen
Falterweg 4
W-3008 Schloss Ricklingen (DE)
Erfinder: Feicho, Lutz
Kantstrasse 20
W-3167 Burgdorf (DE)
Erfinder: Sobe, Alwin
Humperdinck-Strasse 13
W-3202 Sarstedt (DE)
Erfinder: Wischniewski, Martin
Kottowitzer Strasse 24
W-3057 Neustadt a. Rbg. (DE)

(74) Vertreter: Lauer, Dieter, Dr.
c/o Kali-Chemie Aktiengesellschaft Postfach
220
W-3000 Hannover 1 (DE)

## Beschreibung

Die vorliegende Erfindung betrifft eine Arzneimittelformulierung für eine Gelatine-Beißkapsel oder ein Sublingual-Spray, im wesentlichen enthaltend ein Gemisch aus einem coronaraktiven Wirkstoff und einem Alkylenglycoläther.

Eine gattungsgemäße Arzneimittelformulierung ist bereits aus GB-A 970 027 bekannt. Darin werden Aerosol-Zubereitungen von Nitroglycerin als coronaraktivem Wirkstoff beschrieben, die den Wirkstoff gelöst in einem niedrigen Alkohol in Kombination mit einem Treibmittel bzw. Treibmittelgemisch enthalten. Aus Sicherheitsaspekten wird — damit bei einer Verdunstung der leichtflüchtigen Lösungs- bzw. Treibmittel keine explosible Anreicherung an Nitroglycerin erfolgen kann — dem Gemisch ein Phlegmatisierungsmittel zugesetzt, das aus der Reihe Polyalkylenglycole oder deren Derivate ausgewählt ist. In einer breiten Aufzählung werden zwar auch Monoalkyläther von Polyalkylenglycolen als Phlegmatisierungsmittel genannt; bevorzugt werden aber Polyalkylenglycole eingesetzt. Ferner ist in den realisierten Ausführungsformen der Anteil an Phlegmatisierungsmittel stets unter 10 Gew.-%, bezogen auf das den Wirkstoff enthaltende Gemisch.

Andere Arzneimittelformulierungen sind aus EP-A-0 143 857 bekannt, nach der weiche Gelatine-Kapseln eine Füllung aus dem coronaraktiven Wirkstoff Nifedipin in einem Gemisch aus einem Tetrahydrofurfurylalkohol-Polyäthylenglycoläther (THFP) und Polyvinylpyrrolidon (PVP) gelöst enthalten. Der Anteil an essentiellem Bestandteil PVP liegt in den realisierten Beispielen bei mindestens 13 Gew-% der Kapselfüllung. Diese Formulierung überwindet damit zwar Nachteile fester Nifedipin-Formulierungen, die quervernetztes, unlösliches PVP (PVPP) enthalten, unterliegt aber ebenfalls der Beschränkung, große Mengen eines zusätzlichen Hilfsmittels in der Kapselfüllung einsetzen zu müssen.

Aus der DE-OS 3 315 805 sind weiterhin Wirkstoffzubereitungen aus einem nichtlipoiden Tensid (Verbindungen mit Ethylenoxidgruppen und lipophilen Resten) in Verbindung mit einem teilweise mit Wasser mischbaren Lösungsmittel (polare Öle) bekannt. Zwar wird als nichtlipoides Tensid auch Diäthylenglycolbutyläther genannt, doch sind solche Tenside bevorzugt, die 10 Äthylenoxidgruppen enthalten und deren lipoiphiler Rest (Esterrest) aus mehr als 6 C-Atomen besteht.

Ferner werden in der EP-A-0 182 635 percutane Wirkstoffzubereitungen von z.B. hypotensiven Wirkstoffen und Vasodilatoren wie z.B. Isorbiddinitrat in einem polaren Lösungsmittel, welches auch Monoalkyläther von Diäthylenglycol umfaßt, beschrieben. Es handelt sich hier jedoch um percutan anzuwendende Zubereitungen, die notwendigerweise als Absorptionsverstärker einen Nikotinsäure- oder Isonicotinsäureester enthalten.

Der vorliegenden Erfindung liegt daher die Aufgabe zugrunde, neue Arzneimittelgemische für eine Gelatine-Beißkapsel oder ein Sublingual-Spray mit einem Gehalt an coronaraktiven Wirkstoffen zur Verfügung zu stellen. Insbesondere sollen diese Mittel von der Zusammensetzung her einfach gestaltet und möglichst wenig Beschränkungen unterworfen sein.

Diese Aufgabe wird gelöst durch die in den Patentansprüchen charakterisierten Arzneimittelformulierungen.

Diese Arzneimittelformulierungen für ein Gelatine-Beißkapsel oder ein Sublingual-Spray, die im wesentlichen ein Gemisch aus einem coronaraktiven Wirkstoff und einem Alkylenglycoläther enthalten, sind dadurch gekennzeichnet, daß der Wirkstoff Nifedipin oder Isosorbidmono- oder -dinitrat und der Alkylenglycoläther Diäthylenglycolmonoäthyläther ist.

Der Anteil an Alkylenglycoläther am Wirkstoff enthaltenden Gemisch liegt zweckmäßig bei > 10 bis 99 Gew.-%, vorzugsweise bei 30 bis 99 Gew.-%.

In einer Variante der Erfindung kommen als Wirkstoffe Isosorbidnitrate in Frage, die als solche an sich bereits bekannt sind, das Isosorbid-5-mononitrat oder das Isosorbid-2,5-dinitrat.

Eine weitere Variante der Erfindung sieht vor, als Wirkstoffe das an sich bekannte Dihydropyridinderivat Nifedipin einzusetzen.

In der weitesten Fassung der Erfindung ist das Gemisch aus Wirkstoff und Diäthylenglycolmonoälthyläther bereits die fertige Arzneimittelformulierung. Dieses Gemisch ist ersichtlich sehr einfach durch Auflösen des Wirkstoffes im Diäthylenglycolmonoäthyläther herstellbar, wobei — im Gegensatz zur vorbekannten Auflösung, z.B. von Nifedipin in viskosen Medien wie Polyäthylenglycol, Polyäthylenglycol/Glycerin etc. — keine Erwärmung während des Lösungsvorganges notwendig ist. Man erhält klare Lösungen, die als solche in üblicher Weise konfektioniert werden können. Im Falle von Nifedipin als Wirkstoff ist dabei für einen ausreichenden Lichtschutz Sorge zu tragen.

Den Lösungen können im Bedarfsfall übliche Formulierungshilfsmittel zugesetzt werden.

Eine Gruppe Zusätze sind z.B. an sich bekannte Aromatisierungsmittel, ätherische Öle, Menthol Süßungsmittel.

Aufgrund der exzellenten Lösungsmittel-Eigenschaft des Diäthylenglycolmonoäthyläthers ist es ferner möglich, andere pharmazeutisch zulässige Lösungsmittel zuzusetzen, wie beispielsweise Polyole, insbesondere Di- oder Triole, Monoalkohole, insbesondere Ethanol. Diese Lösungsmittel werden bevorzugt in Mengen von 1 bis 15 Gew.-%, bezogen auf Gesamtgemisch, eingesetzt. Die Lösung ist sogar gegen den Zusatz von gewissen Mengen an Wasser stabil, ohne daß es dabei zur Ausfällung der Wirkstoffe kommt.

In Fällen, in denen eine viskosere Einstellung der Wirkstoffformulierung erwünscht ist, kann dieses durch Zusatz bekannter polymerer Bindemittel errreicht werden, die größtenteils ausgezeichnet verträglich mit dem Alkylenglycoläther sind.

Weitere mögliche Zusätze sind solche, die das Hydrophilie-Lipophilie-Verhalten der Wirkstofflösung beeinflussen. Solche Zusätze können Glyceride sein, wie Mono- und/oder Diglyceride, vorzugsweise Triglyceride von Fettsäuren mit 8 bis 12-C-Atomen.

In einer besonderen Ausführungsform der Erfindung können die ggf. durch Zusätze modifizierten Lösungen in Sprühflaschen abgefüllt werden und als Inhalationsspray oder vorzugsweise Sublingualspray zur Anwendung gelangen. Die niedrige Viskosität macht die erfindungsgemäßen Lösungen dabei insbesondere geeignet für den Einsatz in Pumpsprühflaschen, da sie sich gut und gleichmäßig versprüchen lassen. Es ist aber auch eine Abfüllung in an sich bekannten Treibgas-betriebenen Sprühflaschen möglich.

Eine andere besondere Variante der Erfindung sieht vor, die ggf. durch Zusätze modifizierten Lösungen in an sich bekannter Weise in weichen Gelatinekapseln, vorzugsweise als Gelatine-Beißkapseln, abzufüllen. Dazu werden die Lösungen z.B. nach dem Scherer-Verfahren mit einem wäßrigen Gel aus Gelatine und Weichmacher umhüllt und getrocknet. Als Weichmacher eignen sich Polyole wie Glycerin oder Polyäthylenglycol. In diesen Fällen kann es sinnvoll sein, einen geringen Weichmacheranteil bereits der zu verkapselnden Lösung zuzusetzen, um spätere Versprödungen der Kapselhülle zu vermeiden.

In Kombination mit der erfindungsgemäßen Formulierung hat es sich als vorteilhaft gezeigt, als Weichmacher Sorbit oder ähnliche Polyole oder insbesondere Oligomere des Glycerins, vorzugsweise Di- oder Triglycerin einzusetzen. Entsprechende Kapseln benötigen nicht zwingend einen separaten Weichmacherzusatz in der Füllung und sind sehr stabil. Diese Weichmacher werden zu etwa 4 bis 40 Gew.-%, bezogen auf Gesamtmasse der fertigen Kapselhülle, eingesetzt. Wird als Wirkstoff Nifedipin eingesetzt, so sollte die Kapselhülle in an sich bekannter Weise mit einem Lichtschutz versehen werden. Dieses kann in einer einfachsten Variante durch eine lichtundurchlässige Umhüllung der Kapsel geschehen.

Es ist aber auch möglich, in an sich bekannter Weise die Kapselhülle selber lichtundurchlässig auszubilden. Dieses kann z.B. durch Zusatz von löslichen Farbstoffen, die im UV- und im kurzwelligen Tageslichtbereich absorbieren erfolgen und/oder durch Zusatz bunter oder unbunter Pigmente, Lösliche Farbstoffe können in einer Variante auch direkt der Wirkstofflösung zugesetzt werden.

Die erfindungsgemäßen Lösungen lassen sich sehr einfach herstellen und können, wie bereits ausgeführt, entweder direkt weiterverarbeitet oder als Stammansatz für die unterschiedlichsten Formulierungen herangezogen werden.

Die nachfolgenden Beispiele sollen die Erfindung näher charakterisieren ohne ihren Umfang zu begrenzen.

Beispiel 1

Zur Herstellung von Weichgelatine-Kapseln wurde einem Stammsatz aus 6,25 g Nifedipin und 91,75 g Diäthylenglycolmonoäthyläther (DME) 2,0 g Glycerin (85%-ig) zugesetzt. Diese Lösung wurde nach dem Scherer-Verfahren in Weichgelatine-Kapseln der Größe 1,3 minims abgefüllt.

Auch nach 1-jähriger Lagerung zeigten diese Kapseln keine Veränderungen wie Versprödungen etc.

Analog wurden Kapseln mit folgenden Rezepturen (Gewichtsteile) für die Kapselfüllung hergestellt.

|  | 1a | 1b | 1c |
|---|---|---|---|
| Nifedipin | 5,0 | 10,0 | 10,0 |
| DME | 103,5 | 170,3 | 207,0 |
| Glycerin (85 %) | 6,5 | 3,7 | 13,0 |
| Wasser | 6,5 | – | 13,0 |
| Na-Sacarin | 0,15 | – | 0,3 |
| Menthol | 0,35 | – | 0,7 |
| Kapselgröße (minims) | 2 | 3 | 4 |

In Kapsel 1b wurde anstelle Glycerin sowohl in der Füllung als auch in der Kapselwand Diglycerin eingesetzt.

Die Kapselwand war durch Zusatz von Titandioxid und Eisenoxidbraun mit einem Lichtschutz versehen.

## Beispiel 2

Folgende Ansätze wurden in Pumpaerosolflaschen mit Dosierventil (1 Hub = 0,06 ml) abgefüllt (Angabe von Gewichtsteilen).

| Nifedipin | 8,4 |
|---|---|
| Isosorbidmononitrat | – |
| DME | 91,25 |
| Menthol | 0,25 |
| Na-Saccharin | 0,1 |

## Patentansprüche

### Patentansprüche für folgende Vertragsstaaten : AT, BE, CH, DE, FR, GB, IT, LI, LU, NL, SE

1. Arzneimittelformulierung für eine Gelatine-Beißkapsel oder ein Sublingual-Sray, im wesentlichen enthaltend ein Gemisch aus einem coronaraktiven Wirkstoff und einem Alkylenglycoläther dadurch gekennzeichnet, daß der Wirkstoff Nifedipin oder Isosorbidmono- oder -dinitrat und der Alkylenglycoläther Diäthylenglykolmonoäthyläther ist.

2. Arzneimittelformulierung gemäß Anspruch 1, dadurch gekennzeichnet, daß der Anteil an Diäthylenglycolmonoäthyläther am Wirkstoff enthaltenden Gemisch > 10 bis 99 Gew.-%, vorzugsweise 30 bis 99 Gew.-%, beträgt.

3. Gelatine-Beißkapsel enthaltend als Füllung eine Formulierung gemäß einem der vorhergehenden Ansprüche.

4. Beißkapsel gemäß Anspruch 3, dadurch gekennzeichnet, daß die Gelatinehülle zusätzlich lichtundurchlässig ausgebildet ist.

5. Sublingual-Spray, enthaltend einen Wirkstoff und einen Diäthylenglycolmonoäthyläther gemäß einem der Ansprüche 1 oder 2.

### Patentansprüche für folgende Vertragsstaaten : GR, ES

1. Verfahren zur Herstellung einer Arzneimittelformulierung für eine Gelatine-Beißkapsel oder ein Sublingual-Spray, im wesentlichen enthaltend ein Gemisch aus einem coronaraktiven Wirkstoff und einem Alkylenglycoläther dadurch gekennzeichnet, daß man als Wirkstoff Nifedipin oder Isosorbidmono- oder -dinitrat und als Alkylenglycoläther Diäthylenglykolmonoäthyläther einsetzt.

2. Verfahren gemäß Anspruch 1, dadurch gekennzeichnet, daß man als Anteil des Diäthylenglycolmonoäthyläter am Wirkstoff enthaltenden Gemisch > 10 bis 99 Gew.-%, vorzugsweise 30 bis 99 Gew.-%, einsetzt.

3. Verfahren zur Herstellung einer Gelatine-Beißkapsel, dadurch gekennzeichnet, daß man als Füllung eine gemäß einem der vorhergehenden Ansprüche hergestellte Formulierung in die Beißkapsel einbringt.

4. Verfahren gemäß Anspruch 3, dadurch gekennzeichnet, daß man die Formulierung in eine Beißkapsel einbringt, deren Gelatinehülle zusätzlich lichtundurchlässig ausgebildet ist.

5. Verfahren zur Herstellung eines Sublingual-Sprays, dadurch gekennzeichnet, daß man eine einen Wirkstoff und einen Diäthylenglycolmonoäthyläther enthaltende, gemäß einem der Ansprüche 1 oder 2 hergestellte Formulierung in eine Sprühflasche einbringt.

## Revendications

### Revendications pour les Etats contractants suivants : AT, BE, CH, DE, FR, GB, IT, LI, LU, NL, SE

1. Formulation de médicament pour une capsule à croquer en gélatine ou un aérosol sublingual, contenant essentiellement un mélange d'un principe actif à activité coronarienne et d'un éther d'alkylèneglycol, caractérisée en ce que le principe actif est la nidéfipine ou le mono- ou le dinitrate d'isosorbide, et que l'éther d'alkylèneglycol est l'éther monoéthylique du diéthylèneglycol.

2. Formulation de médicament selon la revendication 1, caractérisée en ce que le pourcentage d'éther monoéthylique du diéthylèneglycol dans le mélange contenant le principe actif est compris entre > 10 et 99% en poids, de préférence entre 30 et 99% en poids.

3. Capsule à croquer en gélatine contenant comme remplissage une formulation selon l'une des revendications précédentes.

4. Capsule à croquer selon la revendication 3, caractérisée en ce que la gaine de gélatine est en outre opaque à la lumière.

5. Pulvérisation sublinguale contenant un principe actif et un éther monoéthylique du diéthylèneglycol selon l'une des revendications 1 ou 2.

### Revendications pour les Etats contractants suivants : GR, ES

1. Procédé de préparation d'une formulation de médicament pour une capsule à croquer en gélatine ou une pulvérisation sublinguale, contenant essentiellement un mélange d'un principe actif à activité coronarienne et un éther d'alkylèneglycol, caractérisé en ce qu'on utilise comme principe actif de la nifédipine ou du mono- ou dinitrate d'isosorbide, et comme éther d'alkylèneglycol l'éther monoéthylique du diéthylèneglycol.

2. Procédé selon la revendication 1, caractérisé en ce qu'on utilise de > 10 à 99% en poids, de préférence 30 à 99% en poids de l'éther monoéthylique du diéthylèneglycol par rapport au mélange contenant le principe actif.

3. Procédé de préparation d'une capsule à croquer en gélatine, caractérisé en ce qu'on introduit dans la capsule, en tant que remplissage, une formulation préparée selon l'une des revendications précédentes.

4. Procédé selon la revendication 3, caractérisé en ce qu'on introduit la formulation dans une capsule à croquer dont la gaine en gélatine est en outre opaque à la lumière.

5. Procédé de préparation d'une pulvérisation sublinguale, caractérisé en ce qu'on introduit dans un flacon aérosol une formulation contenant un principe actif et un éther monoéthylique du diéthylèneglycol, préparée selon l'une des revendications 1 ou 2.

## Claims

### Claims for the following Contracting States : AT, BE, CH, DE, FR, GB, IT, LI, LU, NL, SE

1. Medicament formulation for a gelatine bite capsule or a sublingual spray, essentially containing a mixture of a coronary-active active substance and an alkylene glycol ether, characterised in that the active substance is nifedipine or isosorbide mononitrate or dinitrate and the alkylene glycol ether is diethylene glycol monoethyl ether.

2. Medicament formulation according to Claim 1, characterised in that the proportion of diethylene glycol monoethyl ether in the mixture containing active substance is > 10 to 99% by weight, preferably 30 to 99% by weight.

3. Gelatine bite capsule containing as filling a formulation according to one of the preceding Claims.

4. Bite capsule according to Claim 3, characterised in that the gelatine shell is additionally made light-impermeable.

5. Sublingual spray, containing an active substance and a diethylene glycol monoethyl ether according to one of Claims 1 or 2.

## Claims for the following Contracting States : GR, ES

1. Method for producing a medicament formulation for a gelatine bite capsule or a sublingual spray, essentially containing a mixture of a coronary-active active substance and an alkylene glycol ether, characterised in that nifedipine or isosorbide mononitrate or dinitrate is used as the active substance and diethylene glycol monoethyl ether is used as the alkylene glycol ether.

2. Method according to Claim 1, characterised in that > 10 to 99% by weight, preferably 30 to 99% by weight, is used as the proportion of the diethylene glycol monoethyl ether in the mixture containing active substance.

3. Method for producing a gelatine bite capsule, characterised in that a formulation produced according to one of the preceding Claims is introduced into the bite capsule as the filling.

4. Method according to Claim 3, characterised in that the formulation is introduced into a bite capsule, the gelatine shell of which is additionally made light-impermeable.

5. Method for producing a sublingual spray, characterised in that a formulation which contains an active substance and a diethylene glycol monoethyl ether and which is produced according to one of Claims 1 or 2 is introduced into a spray bottle.